# EUROPÄISCHE PATENTANMELDUNG

(11) **EP 4 311 571 A1**
(43) Veröffentlichungstag der Anmeldung: **31.01.2024**
(21) Anmeldenummer: 22187820.0
(22) Anmeldetag: 29.07.2022
(51) Int. Cl.: A61M 60/178, A61M 60/221, A61M 60/232, A61M 60/422, A61M 60/804, A61M 60/82, A61M 60/822

(54) **ROTOR UND FLUIDPUMPE ZUR HERZUNTERSTÜTZUNG**

(71) Anmelder: Berlin Heart GmbH, 12247 Berlin (DE)
(72) Erfinder: Palamarchuk, Dr. Evgenii, 10551 Berlin (DE); Schmidt, Bodo, 14513 Teltow (DE)
(74) Vertreter: Pfenning, Meinig & Partner mbB

(57) **Zusammenfassung**

Die Anmeldung betrifft eine Fluidpumpe (100) zur Herzunterstützung und einen Rotor (200, 200A, 200B, 200C, 200D) für eine solche Fluidpumpe (100). Der Rotor umfasst eine Rotormagnetanordnung (210) zum berührungsfreien Lagern des Rotors (200, 200A, 200B, 200C, 200D) innerhalb einer Kavität (310) der Fluidpumpe (100) derart, dass der Rotor (200, 200A, 200B, 200C, 200D) zum Fördern eines Fluid von einem Einlass (311) der Kavität (310) zu einem Auslass (312) der Kavität (310) um eine Rotationsachse (400) drehbar ist und ein erstes Ende (201) des Rotors (200, 200A, 200B, 200C, 200D) näher an dem Einlass (311) angeordnet ist als ein dem ersten Ende (201) entlang der Rotationsachse (400) entgegengesetztes zweites Ende (202) des Rotors (200, 200A, 200B, 200C, 200D), eine am ersten Ende (201) angeordnete, um die Rotationsachse (400) zentrierte Nabenstruktur (220), die einen oder mehrere Magnete der Rotormagnetanordnung (210) umfasst, eine am zweiten Ende (202) oder zwischen der Nabenstruktur (220) und dem zweiten Ende (202) angeordnete Grundplatte (230) mit einer Beschaufelung (240) zum Fördern des Fluids, wobei Zwischenräume der Beschaufelung (240) Fluidkanäle (241) bilden, die in jeweilige Austrittsöffnungen (242) münden und zum zweiten Ende (202) hin durch die Grundplatte (230) begrenzt sind, wobei mindestens einer, vorzugsweise jeder, der Fluidkanäle (241) eine den Fluidkanal (241) mit der Kavität (310) verbindende Durchlassöffnung (246) aufweist, die sich zwischen der Austrittsöffnung (242) und der Nabenstruktur (220) wenigstens bereichsweise entlang einer dem ersten Ende (201) zugewandten ersten Seite des Fluidkanals (241) erstreckt.

## Beschreibung

Die Anmeldung betrifft einen Rotor für eine Fluidpumpe zur Herzunterstützung sowie eine entsprechende Fluidpumpe. Der Gegenstand der Anmeldung ist insbesondere auf dem Gebiet der kurzfristigen oder langfristigen Herzunterstützung mittels implantierbarer Fluidpumpen, insbesondere VADs (VAD steht dabei für ventricular assist device), einsetzbar.

Nach dem Stand der Technik sind verschiedene Rotationsfluidpumpen zur Herzunterstützung bekannt, insbesondere solche, die als implantierbare und mit einem vorhandenen Herzen fluidisch verbindbare VADs verwendbar sind.

Rotoren solcher Fluidpumpen weisen häufig komplexe Geometrien auf, die entsprechend komplexe Fertigungsverfahren erforderlich machen. Beispielsweise können Rotorgeometrien mit Hohlräumen im Inneren des Rotors eine mehrstückige Fertigung, also eine Fertigung aus mehreren Einzelteilen, und ein Zusammensetzen der Einzelteile und/oder eine nachträgliche Oberflächenbearbeitung zum Sicherstellen hinreichender Oberflächenqualität erforderlich machen. Beispielsweise kann bei einem durch Schweißen mehrstückig gebildeten Rotor eine Nachbearbeitung von Schweißnähten erforderlich sein, die etwa durch eine begrenzte Zugänglichkeit etwaiger Hohlräume erschwert sein kann. Auch können die genannten Aspekte das Einhalten von Fertigungstoleranzen erschweren.

Es ist bei Rotoren von Fluidpumpen ferner wünschenswert, bestimmte Parametervorgaben, etwa hinsichtlich bestimmter Kennlinien des Rotors und/oder der Fluidpumpe, einzuhalten. Etwa kann eine dp-Q-Kennlinie wünschenswert sein, die innerhalb eines vorgegebenen Bereichs verläuft und/oder jedenfalls bereichsweise eine Steilheit aufweist, die größer oder gleich einer vorgegebenen Mindeststeilheit ist. Die dp-Q-Kennlinie beschreibt eine Druckdifferenz (dp) über die Fluidpumpe oder einen, insbesondere den Rotor umfassenden, Teil der Fluidpumpe als Funktion eines Volumenstroms (Q) durch die Fluidpumpe. Durch eine geeignete dp-Q-Kennlinie kann etwa das Auftreten zu geringer oder negativer (d. h. der Pumprichtung entgegengesetzter) Volumenströme vermieden oder verringert werden. Auch kann durch eine geeignete dp-Q-Kennlinie beispielsweise eine zuverlässige Schätzung des Volumenstroms unter Verwendung einer gemessenen oder aus Messdaten bestimmten Druckdifferenz ermöglicht werden, was für eine Steuerung oder Regelung der Fluidpumpe, beispielsweise unter Verwendung einer Nullkraftregelung der weiter unten beschriebenen Art, vorteilhaft sein kann. Ferner kann beispielsweise eine Axialkraftkennlinie wünschenswert sein, die innerhalb eines vorgegebenen Bereichs verläuft und/oder wenigstens bereichsweise monoton, vorzugsweise streng monoton und/oder möglichst steil, verläuft. Die Axialkraftkennlinie beschreibt eine auf den entlang einer Rotationsachse wirkende Axialkraft als Funktion eines Volumenstroms durch die Fluidpumpe. Durch eine geeignete Axialkraftkennlinie kann beispielsweise eine zuverlässige Schätzung des Volumenstroms unter Verwendung einer gemessenen oder aus Messdaten bestimmten Axialkraft und/oder Axialposition ermöglicht werden, was wiederum für eine Steuerung oder Regelung der Fluidpumpe, beispielsweise unter Verwendung einer Nullkraftregelung, vorteilhaft sein kann.

Vor dem Hintergrund des Stands der Technik liegt der vorliegenden Anmeldung die Aufgabe zugrunde, einen Rotor für eine Fluidpumpe zur Herzunterstützung sowie eine entsprechende Fluidpumpe bereitzustellen, die wenigstens einige der genannten erwünschten Eigenschaften aufweisen.

Die Aufgabe wird gelöst, indem ein Rotor gemäß Anspruch 1 und eine Fluidpumpe zur Herzunterstützung gemäß Anspruch 14 vorgeschlagen werden. Weiterbildungen und bevorzugte Ausgestaltungen ergeben sich mit den Merkmalen gemäß den jeweiligen Unteransprüchen.

Der vorgeschlagene Rotor für eine Fluidpumpe zur Herzunterstützung umfasst
eine Rotormagnetanordnung zum berührungsfreien Lagern des Rotors innerhalb einer Kavität der Fluidpumpe derart, dass der Rotor zum Fördern eines Fluid von einem Einlass der Kavität zu einem Auslass der Kavität um eine Rotationsachse drehbar ist und ein erstes Ende des Rotors näher an dem Einlass angeordnet ist als ein dem ersten Ende entlang der Rotationsachse entgegengesetztes zweites Ende des Rotors,
eine am ersten Ende angeordnete, um die Rotationsachse zentrierte Nabenstruktur, die einen oder mehrere Magnete der Rotormagnetanordnung umfasst,
eine am zweiten Ende oder zwischen der Nabenstruktur und dem zweiten Ende angeordnete Grundplatte mit einer Beschaufelung zum Fördern des Fluids, wobei Zwischenräume der Beschaufelung Fluidkanäle bilden, die in jeweilige Austrittsöffnungen münden und zum zweiten Ende hin durch die Grundplatte begrenzt sind.

Die Fluidpumpe (auch kurz als Pumpe bezeichnet) ist eine Rotationsfluidpumpe, die insbesondere eine Zentrifugalpumpe, alternativ auch eine Axialpumpe oder eine Mischform (also etwa als Pumpe, bei der das geförderte Fluid im Betrieb mit sowohl axialen als auch radialen Strömungskomponenten aus dem Auslass der Kavität austritt) sein kann. Der Rotor und die Pumpe sind insbesondere zum Fördern von Blut als Fluid eingerichtet, wozu der Einlass und der Auslass der Kavität fluidisch mit einem Herzen und/oder mit einem mit dem Herzen verbundenen Blutgefäß verbindbar oder verbunden sind.

Der Rotor ist in der Kavität vorzugsweise allseitig berührungsfrei magnetisch lagerbar; zusätzlich zu magnetischen Kräften können andere Arten von Kräften, etwa hydrodynamische Kräfte, zu dem Lagern des Rotors in der Kavität beitragen.

Die Rotormagnetanordnung ist dazu eingerichtet, zum Lagern des Rotors in der Kavität mit einer Statormagnetlageranordnung der Fluidpumpe magnetisch zu wechselwirken. Insbesondere können die Rotormagnetanordnung und die Statormagnetlageranordnung ein aktives Magnetlager entlang wenigstens einer ersten Richtung bilden, d. h. ein Magnetlager, bei dem ein Magnetfeld durch Ansteuern von Elektromagneten entlang der ersten Richtung zum Ausüben einer variierbaren Lagerkraft auf den Rotor variierbar ist. Alternativ können die Rotormagnetanordnung und die Statormagnetlageranordnung ein passives Magnetlager entlang der ersten Richtung bilden. Die Rotormagnetanordnung und die Statormagnetlageranordnung können dazu eingerichtet sein, den Rotor entlang weiterer Raumrichtungen passiv magnetisch oder ebenfalls aktiv magnetisch zu lagern.

Der Rotor ist dazu eingerichtet, zusammen mit einem Stator der Fluidpumpe einen Elektromotor zu bilden, so dass der Rotor zum Fördern des Fluids um die Rotationsachse in Rotation versetzbar ist. Insbesondere können Permanentmagnete der Rotormagnetanordnung zusammen mit Spulen der Statormagnetlageranordnung und/oder Spulen einer Statormotoranordnung einen Motor, etwa einen bürstenlosen Gleichstromotor, bilden. Der Stator und der Rotor können insbesondere einen Axialflussmotor bilden; alternativ können der Stator und der Rotor beispielsweise einen Radialflussmotor bilden.

Durch die am ersten Ende angeordnete Nabenstruktur kann eine besonders stabile Magnetlagerung des Rotors in der Kavität erzielbar sein, wobei der oder die von der Nabenstruktur umfassten Magnete der Rotormagnetanordnung u. a. gegen Verkippen des Rotors bezüglich der Rotationsachse stabilisierend wirken können.

Die tatsächliche Rotationsachse kann, insbesondere im Betrieb, entsprechend einer Bewegung des Rotors verschoben und/oder verkippt werden. Bei der Beschreibung von Anordnungen und Orientierungen von Elementen hinsichtlich der Rotationsachse wird aber im Folgenden angenommen, dass die Rotationsachse feststeht und einer festen Gehäuse-Längsachse der Pumpe entspricht. Die Nabenstruktur kann in Richtung des ersten Endes von der Grundplatte hervorstehen. Eine von der Grundplatte hervorstehende Länge der Nabenstruktur entlang der Rotationsachse kann beispielsweise wenigstens ein Viertel, wenigstens ein Drittel oder wenigstens die Hälfte einer Gesamtlänge des Rotors entlang der Rotationsachse betragen; insbesondere kann die von der Grundplatte hervorstehende Länge der Nabenstruktur entlang der Rotationsachse zwischen 40 Prozent und 45 Prozent oder zwischen 35 Prozent und 40 Prozent der Gesamtlänge des Rotors entlang der Rotationsachse betragen. Eine äußere Breite der Nabenstruktur senkrecht zur Rotationsachse kann beispielsweise wenigstens ein Drittel oder wenigstens die Hälfte, insbesondere ca. 50 Prozent, einer Gesamtbreite des Rotors senkrecht zur Rotationsachse betragen. Die Nabenstruktur ist vorzugsweise bezüglich der Rotationsachse im Wesentlichen rotationssymmetrisch geformt. Die Nabenstruktur kann insbesondere wenigstens abschnittsweise im Wesentlichen zylindrisch und/oder wenigstens abschnittsweise im Wesentlichen konisch geformt sein; die Nabenstruktur kann einen lang der Rotationsachse variablen Querschnitt aufweisen.

Die Nabenstruktur kann einen oder mehrere Hohlräume zum Aufnehmen des oder der von der Nabenstruktur umfassten Magnete der Rotormagnetanordnung umfassen. Einer oder mehrere des einen oder der mehreren von der Nabenstruktur umfassten Magnete der Rotormagnetanordnung können dazu eingerichtet sein, mit einer Steuerspule der Statormagnetlageranordnung zum Ausüben einer Kraft, insbesondere einer Kraft entlang der Rotationsachse, auf den Rotor zusammenzuwirken. Einer oder mehrere des einen oder der mehreren von der Nabenstruktur umfassten Magnete der Rotormagnetanordnung können dazu eingerichtet sein, mit einer Motorspule der Statormagnetlageranordnung und/oder einer Statormotoranordnung zum Ausüben eines Drehmoments in Bezug auf die Rotationsachse auf den Rotor und/oder zum Ausüben einer Kraft, insbesondere einer Kraft entlang der Rotationsachse, auf den Rotor zusammenzuwirken.

Die Grundplatte kann im Wesentlichen scheibenförmig sein, beispielsweise kann die Grundplatte im Wesentlichen die Form eines abgeflachten Zylinders aufweisen. Die Grundplatte kann einen oder mehrere weitere Magnete der Rotormagnetanordnung umfassen. Die Grundplatte kann einen oder mehrere Hohlräume zum Aufnehmen des oder der von der Grundplatte umfassten Magnete der Rotormagnetanordnung umfassen. Einer oder mehrere des einen oder der mehreren von der Grundplatte umfassten Magnete der Rotormagnetanordnung können dazu eingerichtet sein, mit einer Steuerspule der Statormagnetlageranordnung zum Ausüben einer Kraft, insbesondere einer Kraft entlang der Rotationsachse, auf den Rotor zusammenzuwirken. Einer oder mehrere des einen oder der mehreren von der Grundplatte umfassten Magnete der Rotormagnetanordnung können dazu eingerichtet sein, mit einer Motorspule der Statormagnetlageranordnung zum Ausüben eines Drehmoments in Bezug auf die Rotationsachse auf den Rotor und/oder zum Ausüben einer Kraft, insbesondere einer Kraft entlang der Rotationsachse, auf den Rotor zusammenzuwirken.

Die Beschaufelung umfasst vorzugsweise eine Mehrzahl von Schaufeln, wobei die Fluidkanäle zwischen einander zugewandten jeweiligen Seitenflächen benachbarter Schaufeln ausgebildet sind. Die Schaufeln können im Wesentlichen radial von einem zentralen Bereich der Grundplatte zu einem äußeren Bereich der Grundplatte hin verlaufen, wobei die Schaufeln vorzugsweise in Bezug auf die Radialrichtung angewinkelt und/oder spiralartig gekrümmt sind. Die Beschaufelung kann eingerichtet sein, das geförderten Fluid im Betrieb mit einer radialen Strömungskomponente (in Richtung der Austrittsöffnung) zu beaufschlagen. Die Schaufeln können in Bezug auf die Rotationsachse angewinkelt und/oder gekrümmt sein.

Es ist vorgesehen, dass mindestens einer der Fluidkanäle eine den Fluidkanal mit der Kavität verbindende Durchlassöffnung aufweist, die sich zwischen der Austrittsöffnung und der Nabenstruktur wenigstens bereichsweise entlang einer dem ersten Ende zugewandten ersten Seite des Fluidkanals erstreckt.

Die somit gegebene, zum ersten Ende hin zumindest teilweise (im Bereich der Durchlassöffnungen) offene, zum zweiten Ende hin geschlossene (durch die Grundplatte begrenzte) Geometrie der Fluidkanäle wird hier auch als halboffene Rotorgeometrie, der entsprechende Rotor als halboffener Rotor bezeichnet. Eine auch zum ersten Ende hin geschlossene (etwa durch eine der Grundplatte gegenüberliegende Deckplatte begrenzte) Geometrie der Fluidkanäle wird hier auch als geschlossene Rotorgeometrie, der entsprechende Rotor als geschlossener Rotor bezeichnet.

Indem solche Durchlassöffnungen vorgesehen werden, kann eine Fertigung des Rotors vereinfacht und/oder das Einhalten von vorgegebenen Fertigungstoleranzen erleichtert werden. Insbesondere kann auf diese Weise eine einstückige Fertigung einer Komponente des Rotors ermöglicht werden, die bei Fehlen der jeweiligen Durchlassöffnung mehrstückig zu fertigen wäre. Insbesondere können Teilungen bzw. Nähte im Bereich der Beschaufelung vermieden werden. Durch die im Bereich der Durchlassöffnung zum ersten Ende hin offene Rotorgeometrie kann etwa ein einstückiges Fräsen oder Gießen der Grundplatte oder jedenfalls eines Teils der Grundplatte zusammen mit der Beschaufelung bzw. einem Teil der Beschaufelung erfolgen. Auch kann diese zum ersten Ende hin offene Rotorgeometrie einen Zugang für ein Nachbearbeiten, etwa Polieren oder Schleifen, von Oberflächen des Rotors schaffen, insbesondere im Bereich der Fluidkanäle.

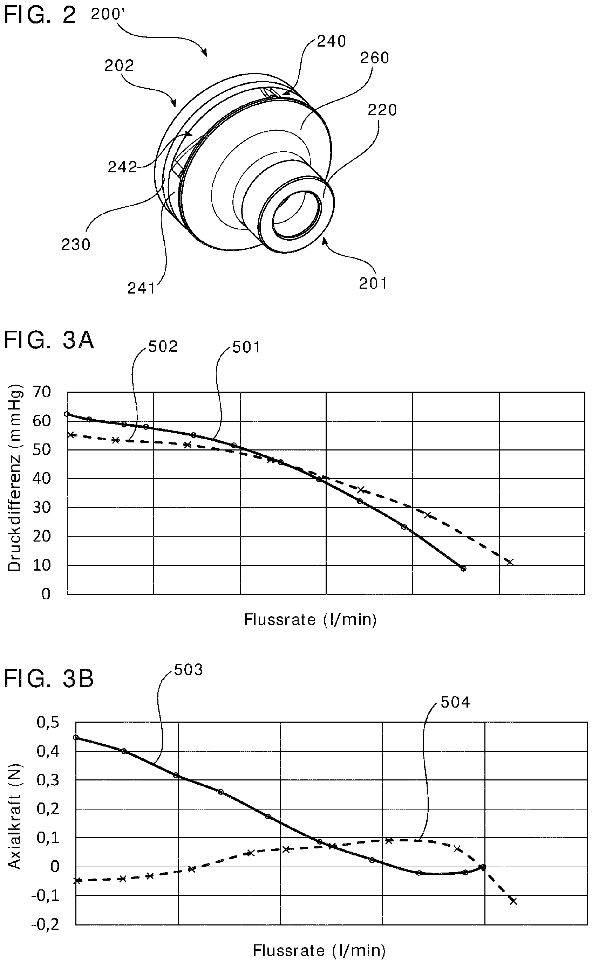

Auch stellt das Vorsehen der Durchlassöffnungen einen Parameterraum für die Konstruktion des Rotors bzw. der Fluidpumpe zur Verfügung, innerhalb dessen bestimmte Parametervorgaben erreicht und/oder Betriebsparameter der Fluidpumpe gegenüber anderen Rotorgeometrien angepasst und/oder optimiert werden können. Etwa hat sich gezeigt, dass eine Steilheit einer dp-Q-Kennlinie bei einem Rotor mit Durchlassöffnungen, also mit einer zum ersten Ende hin offenen Rotorgeometrie, über bestimmte Bereiche erhöht sein kann gegenüber einem Rotor mit ansonsten vergleichbarer Konstruktion, aber ohne Durchlassöffnungen, also mit zum ersten Ende hin geschlossener Rotorgeometrie (vgl. etwa FIG. 3A und deren Beschreibung weiter unten). Auch hat sich gezeigt, dass eine Axialkraftkennlinie bei einem Rotor mit zum ersten Ende hin offener Rotorgeometrie gegenüber einem Rotor mit ansonsten vergleichbarer Konstruktion, aber mit zum ersten Ende hin geschlossener Rotorgeometrie, bereichsweise steiler und über einen größeren Bereich monoton verlaufen kann (vgl. etwa FIG. 3B und deren Beschreibung weiter unten).

Vorzugsweise weisen mehrere in regelmäßigen Abständen um die Rotationsachse herum angeordnete Fluidkanäle, insbesondere alle Fluidkanäle, jeweils eine Durchlassöffnung der beschriebenen Art auf.

Es kann vorgesehen sein, dass sich die Durchlassöffnung mindestens eines, vorzugsweise jedes, der Fluidkanäle entlang wenigstens eines Teils, insbesondere entlang nur eines Teils, einer Ausdehnung des Fluidkanals von der Austrittsöffnung in Richtung der Nabenstruktur erstreckt. Es kann vorgesehen sein, dass sich die Durchlassöffnung mindestens eines, vorzugsweise jedes, der Fluidkanäle, entlang der gesamten Ausdehnung des Fluidkanals von der Austrittsöffnung bis zu der Nabenstruktur erstreckt. Indem angepasst wird, in welchem Umfang sich die Durchlassöffnungen entlang der Ausdehnung der Fluidkanäle erstrecken, können Betriebsparameter der Pumpe und/oder des Rotors innerhalb des o. g. Parameterraums angepasst und/oder gegeneinander sowie im Hinblick auf die erzielbare Vereinfachung der Rotorfertigung abgewogen werden. Erstreckt sich die Durchlassöffnung nur entlang eines Teils der genannten Ausdehnung des Fluidkanals, kann beispielsweise eine mögliche Blutschädigung im Betrieb und/oder eine Anfälligkeit der Rotorposition gegenüber extern wirkenden Drehmomenten und/oder durch externe Einflüsse verursachten Bewegungen der Pumpe (etwa Drehungen des Patienten) begrenzt werden.

Eine entsprechende Wirkung kann auch durch eine teilweise Begrenzung der Fluidkanäle zum ersten Ende hin mittels Vorsprüngen der Beschaufelung (auch als Winglets bezeichnet) erzielt werden. So kann mindestens einer, vorzugsweise jeder, der Fluidkanäle an der ersten Seite auf Höhe der Durchlassöffnung teilweise durch einen Vorsprung einer an den jeweiligen Fluidkanal angrenzenden Fläche der Beschaufelung begrenzt sein. Der Vorsprung kann entgegen einer Rotationsrichtung des Rotors von der an den jeweiligen Fluidkanal angrenzenden Fläche der Beschaufelung auskragen und/oder diese Fläche kann eine konkave Krümmung aufweisen. Die genannten Wirkungen des Vorsprungs können in diesem Fall besonders ausgeprägt sein. Der Vorsprung kann in einer Rotationsrichtung des Rotors von der an den jeweiligen Fluidkanal angrenzenden Fläche der Beschaufelung auskragen und/oder diese Fläche kann eine konvexe Krümmung aufweisen. In diesem Fall kann etwa eine Fertigung des Rotors aufgrund der guten Zugänglichkeit der konvex gekrümmten Fläche verhältnismäßig einfach sein.

Die Nabenstruktur kann einen entlang der Rotationsachse verlaufenden und am ersten Ende offenen Eintrittskanal umfassen, der mit den Fluidkanälen innerhalb des Rotors fluidisch verbunden ist. Die Nabenstruktur kann somit als Fluideinlass des Rotors wirken, durch den das durch den Einlass der Kavität eintretende Fluid zu der Beschaufelung geleitet wird. Insbesondere können die Fluidkanäle in den Eintrittskanal mündende Eintrittsöffnungen aufweisen.

Ein Übergang von der Beschaufelung zu der Nabenstruktur kann verrundet sein. Ein verrundeter Übergang kann aus fertigungstechnischer Sicht zu bevorzugen sein und/oder eine verbessertes hydrodynamisches Verhalten, etwa verringerte Turbulenzen und/oder verringerte Blutschädigung, bewirken.

Eine die Beschaufelung umfassende Komponente des Rotors kann einstückig, insbesondere durch Fräsen aus einem zusammenhängenden Werkstoffstück, gebildet sein. Die die Beschaufelung umfassende einstückig gebildete Komponente kann wenigstens einen Teil der Grundplatte und/oder wenigstens einen Teil der Nabenstruktur umfassen. Die Nabenstruktur kann als mit der die Beschaufelung umfassenden Komponente verbindbare oder verbundene, separate Nabenbaugruppe gebildet sein. Die Nabenstruktur und die die Beschaufelung umfassende Komponente können zueinander komplementäre Ausrichtelemente umfassen, die etwa eine Flanschverbindung und/oder eine Nutverbindung und/oder eine Stiftverbindung bilden können.

Der Rotor kann aus einer ersten Komponente, umfassend wenigstens einen Teil der Nabenstruktur, und mindestens einer zweiten Komponente zusammengesetzt oder zusammensetzbar sein. Der Rotor kann aus der genannten ersten Komponente, einer zweiten Komponente, umfassend wenigstens einen weiteren Teil der Nabenstruktur, und mindestens einer dritten Komponente, umfassend wenigstens einen Teil der Grundplatte, zusammengesetzt oder zusammensetzbar sein.

Die Grundplatte kann eine zentrale Ausnehmung zur berührungsfreien Aufnahme eines Teils des Stators, insbesondere eines Zapfens des Stators, aufweisen. DieserTeil bzw. Zapfen kann eine Magnetlagerkomponente, insbesondere eine oder mehrere Magnete der Statormagnetlageranordnung, der Fluidpumpe umfassen. Von der Grundplatte umfasste Magnete der Rotormagnetanordnung können peripher zu der zentralen Ausnehmung angeordnet sein.

Die vorgeschlagene Fluidpumpe zur Herzunterstützung umfasst
einen Rotor der oben vorgeschlagenen Art und
einen Stator mit einer Kavität zur Aufnahme des Rotors und einer Statormagnetlageranordnung, die zusammen mit der Rotormagnetanordnung ein Magnetlager, insbesondere ein aktives Magnetlager, zum berührungsfreien Lagern des Rotors innerhalb der Kavität bildet.

Der vorgeschlagene Rotor entfaltet in der vorgeschlagenen Fluidpumpe seine oben genannten Wirkungen und Vorteile. Die Fluidpumpe kann entsprechend den vorbeschriebenen optionalen Merkmalen des Rotors weiter ausgestaltet werden. Der Rotor kann Merkmale umfassen, die optionalen Merkmalen der Fluidpumpe entsprechen.

Die Pumpe kann eine Steuereinheit zum Steuern und/oder Regeln einer Rotation und/oder Position des Rotors innerhalb der Kavität umfassen und/oder mit einer solchen Steuereinheit verbindbar oder verbunden sein. Die Pumpe kann einen oder mehrere Sensoren zum Bereitstellen von Messsignalen, entsprechend Parametern der Rotation und/oder Position des Rotors und/oder entsprechend anderen Parametern, etwa einem Druck und/oder einer Druckdifferenz und/oder auf den Rotor wirkenden Kräften und/oder Drehmomenten. Die Steuereinheit kann dazu eingerichtet sein, die Rotation und/oder Position des Rotors basierend auf den Messsignalen zu steuern bzw. zu regeln.

Die Steuereinheit kann beispielsweise dazu eingerichtet sein, ein an den Motorspulen abgreifbares Messsignal mit einem von der Position und/oder Rotation des Rotors abhängigen Anteil zu erfassen; der Anteil kann insbesondere einer aufgrund der Rotation des Rotors induzierten elektromotorischen Gegenkraft (Back Electromotive Force, Back-EMF) entsprechen. Auf diese Weise ist eine Bereitstellung eines Messsignals, dass insbesondere für die Steuerung und/oder Regelung der Rotation und/oder Position des Rotors verwendbar ist, ohne zusätzliche Sensoren möglich.

Ferner kann die Steuereinheit beispielsweise dazu eingerichtet sein, den Rotor auf eine (insbesondere ortsveränderliche) Ziel-Lagerposition einzuregeln, an der auf den Rotor wirkende äußere Kräfte entlang einer Lagerwirkrichtung, etwa entlang der Rotationsachse, sich zu einer vorgegebenen Kraft, insbesondere einer Nullkraft, addieren und/oder an der eine zum Ansteuern der Steuer- und/oder Motorspulen aufgewandte Leistung minimal ist. Durch eine solche Regelung, die auch als Nullkraftregelung bezeichnet wird, ist ein energieeffizienter Betrieb der Pumpe möglich.

Ausführungsbeispiele des Anmeldungsgegenstandes werden nachfolgend mit Bezugnahme auf Zeichnungen erläutert. Dabei zeigen, jeweils schematisch:
FIG. 1A einen Längsschnitt einer Fluidpumpe gemäß einem Beispiel,
FIG. 1B und FIG. 1C perspektivische Ansichten eines Rotors der in FIG. 1A gezeigten Fluidpumpe,
FIG. 2 einen perspektivische Ansicht eines nicht dem Anmeldungsgegenstand entsprechenden Rotors,
FIG. 3A und FIG. 3B verschiedene Kennlinien von Fluidpumpen mit Rotoren der in FIG. 1A-1C und FIG. 2 gezeigten Arten,
FIG. 4A und FIG. 4B perspektivische Ansichten eines Rotors gemäß einem weiteren Beispiel,
FIG. 5A und FIG. 5B perspektivische Ansichten eines Rotors gemäß einem weiteren Beispiel,
FIG. 6A eine perspektivische Ansicht von Baugruppen eines Rotors gemäß einem weiteren Beispiel,
FIG. 6B eine weitere perspektivische Ansicht einer der in FIG. 6A gezeigten Baugruppen,
FIG. 7A eine perspektivische Ansicht von Baugruppen eines Rotors gemäß einem weiteren Beispiel,
FIG. 7B eine weitere perspektivische Ansicht einer der in FIG. 7A gezeigten Baugruppen.

Wiederkehrende und ähnliche Merkmale verschiedener Ausführungsformen sind in den Zeichnungen mit identischen alphanumerischen Bezugszeichen versehen. Bereits im Zusammenhang mit einer anderen Zeichnung erklärte oder gezeigte Bezugszeichen können dabei ausgelassen werden.

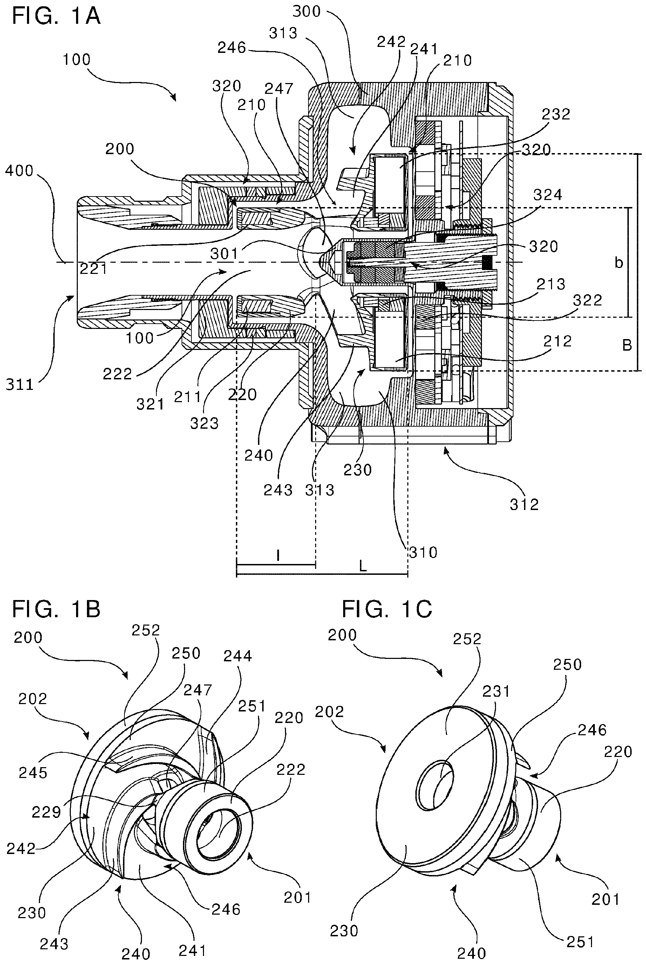

Die in FIG. 1A gezeigte Fluidpumpe 100 umfasst einen Rotor 200 (in FIG. 1B und FIG. 1C in weiteren Ansichten gezeigt) sowie einen als Gehäuse ausgebildeten Stator 300. Der Stator 300 umfasst eine Kavität 310 zur Aufnahme des Rotors 200.

Der Rotor 200 umfasst eine Rotormagnetanordnung 210 zum berührungsfreien Lagern des Rotors 200 innerhalb der Kavität 310 derart, dass der Rotor 200 zum Fördern eines Fluid von einem Einlass 311 der Kavität zu einem Auslass 312 der Kavität um eine Rotationsachse 400 drehbar ist und ein erstes Ende 201 des Rotors 200 näher an dem Einlass 311 angeordnet ist als ein dem ersten Ende 201 entlang der Rotationsachse 400 entgegengesetztes zweites Ende 202 des Rotors 200. Näher an dem ersten Ende 201 bzw. dem Einlass 311 als an dem zweiten Ende 202 bzw. dem Auslass 312 angeordnete Merkmale der Fluidpumpe 100 werden auch als einlassseitige Merkmale, näher an dem zweiten Ende 202 bzw. dem Auslass 312 als an dem ersten Ende 201 bzw. dem Einlass 311 angeordnete Merkmale der Fluidpumpe 100 auch als auslassseitige Merkmale bezeichnet.

Der Rotor 200 umfasst ferner eine am ersten Ende 201 angeordnete, um die Rotationsachse 400 zentrierte Nabenstruktur 220, die einen oder mehrere einlassseitige Rotorlagermagnete 211 der Rotormagnetanordnung 210 umfasst. Beispielsweise können ein oder mehrere ringförmige und/oder segmentierte einlassseitige Rotorlagermagnete 211 vorgesehen sein.

Der Rotor 200 umfasst weiterhin eine am zweiten Ende 202 angeordnete Grundplatte 230 mit einer Beschaufelung 240 zum Fördern des Fluids, wobei Zwischenräume der Beschaufelung Fluidkanäle 241 bilden, die in jeweilige Austrittsöffnungen 242 münden und zum zweiten Ende 202 hin durch die Grundplatte 230 begrenzt sind. Die Grundplatte 230 kann alternativ zwischen der Nabenstruktur 220 und dem zweiten Ende 202 angeordnet sein. Die Grundplatte 230 umfasst eine zentrale Ausnehmung 231 zur berührungsfreien Aufnahme eines Zapfens 301 des Stators 300.

Die Fluidpumpe 100 ist eine Rotationsfluidpumpe, die als Zentrifugalpumpe ausgeführt ist. Alternativ kann die Fluidpumpe auch eine Axialpumpe oder eine Mischform sein. Der Rotor 200 und die Fluidpumpe 100 sind zum Fördern von Blut als Fluid eingerichtet, wozu der Einlass 311 und der Auslass 312 der Kavität 310 fluidisch mit einem Herzen und/oder mit einem mit dem Herzen verbundenen Blutgefäß verbindbar oder verbunden sind. Die Kavität 310 umfasst eine Volute 313, in der aus den Fluidkanälen 241 austretendes Fluid gesammelt und dem Auslass 312 zugeführt wird.

Der Rotor 200 ist in der Kavität 300 allseitig berührungsfrei magnetisch lagerbar; zusätzlich zu magnetischen Kräften können andere Arten von Kräften, etwa hydrodynamische Kräfte, zu dem Lagern des Rotors 200 in der Kavität 300 beitragen.

Der Stator 300 umfasst eine Statormagnetlageranordnung 320, die zusammen mit der Rotormagnetanordnung 210 ein Magnetlager zum berührungsfreien Lagern des Rotors innerhalb der Kavität bildet. Die Rotormagnetanordnung 210 ist entsprechend dazu eingerichtet, zum Lagern des Rotors 200 in der Kavität mit der Statormagnetlageranordnung 320 magnetisch zu wechselwirken. Insbesondere bilden die Rotormagnetanordnung 210 und die Statormagnetlageranordnung 320 ein aktives Magnetlager entlang der Rotationsachse 400. Die Rotormagnetanordnung 210 und die Statormagnetlageranordnung 320 sind ferner dazu eingerichtet, den Rotor 200 in Richtungen senkrecht zur Rotationsachse 400 passiv magnetisch zu lagern; alternativ kann auch eine passive Magnetlagerung entlang der Rotationsachse und/oder eine aktiv Magnetlagerung in dazu senkrechten Richtungen vorgesehen sein. Im beschriebenen Beispiel umfasst die Rotormagnetanordnung 210 Permanentmagnete, die Statormagnetlageranordnung 320 umfasst sowohl Spulen aufweisende Elektromagnete als auch Permanentmagnete. Es können auch andere Kombinationen von Permanentmagneten und/oder induzierbaren Magneten und/oder Elektromagneten vorgesehen sein.

Der Rotor 200 ist dazu eingerichtet, zusammen mit dem Stator 300 der Fluidpumpe 100 einen bürstenlosen Gleichstrommotor zu bilden, so dass der Rotor 200 zum Fördern des Fluids um die Rotationsachse 400 in Rotation versetzbar ist. Hierzu umfasst die Rotor Der Stator 200 und der Rotor 300 bilden einen Axialflussmotor; alternativ können der Stator und der Rotor einen anderen Motortyp, etwa einen Radialflussmotor bilden.

Der oder die einlassseitigen Rotorlagermagnete 211 sind dazu eingerichtet, mit mindestens einer einlassseitig angeordneten Steuerspule 321 der Statormagnetlageranordnung 320 zum Ausüben einer Kraft entlang der Rotationsachse 400 auf den Rotor 200 zusammenzuwirken (Teil des aktiven axialen Magnetlagers), wobei die Kraft je nach Bestromung der Steuerspule 321 anziehend oder abstoßend wirken kann. Die Rotormagnetanordnung 210 umfasst ferner einen oder mehrere auslassseitige Rotormotormagnete 212, die dazu eingerichtet sind, mit auslassseitig angeordneten Motorspulen 322 der Statormagnetlageranordnung 320 zum Ausüben eines Drehmoments bezüglich der Rotationsachse 300 auf den Rotor 200 zusammenzuwirken (Teil des Axialflussmotors). Es kann alternativ oder zusätzlich eine separate Statormotoranordnung mit Motorspulen vorgesehen sein. Die Rotormotormagnete 212 können zusätzlich zum Zusammenwirken mit den Motorspulen 322 zum Ausüben einer Kraft entlang der Rotationsachse 400 eingerichtet sein (Teil des aktiven axialen Magnetlagers), wobei die Kraft je nach Bestromung der Motorspulen 322 anziehend oder abstoßend wirken kann.

Der oder die einlassseitigen Rotorlagermagnete 211 bilden zusammen mit einem oder mehreren einlassseitigen Statorlagermagneten 323 der Statormagnetlageranordnung 320 ein einlassseitiges passives Radialmagnetlager. Die Rotormagnetanordnung 210 umfasst ferner einen oder mehrere auslassseitige Rotorlagermagnete 213, die zusammen mit einem oder mehreren auslassseitigen Statorlagermagneten 324 der Statormagnetlageranordnung 320 ein auslassseitiges passives Radialmagnetlager bilden. Der oder die auslassseitigen Rotorlagermagnete 213 sind peripher zu der zentralen Ausnehmung 231 der Grundplatte 230 angeordnet. Der Zapfen 301 umfasst den oder die auslassseitigen Statorlagermagnete 324. Durch die Anordnung der beiden Radialmagnetlager in der Nabenstruktur 220 und in der Grundplatte 230 können die Radialmagnetlager u. a. gegen Verkippen des Rotors bezüglich der Rotationsachse stabilisierend wirken.

Die beschriebene Anordnung und Kombination der Magnete der Rotormagnetanordnung 210 und der Statormagnetlageranordnung 320 sind exemplarisch. Andere Anordnungen und/oder Kombinationen und/oder das Hinzufügen und/oder Weglassen von Magneten sind möglich. Beispielsweise kann der Motor einlassseitig statt, wie hier, auslassseitig angeordnet sein. Die Steuerspule kann auslassseitig angeordnet sein. Die beiden Radialmagnetlager können in einer gemeinsamen Ebene, etwa innenliegend und außenliegend an der Grundplatte, angeordnet sein statt, wie hier, axial beabstandet zu sein, oder es kann zusätzlich eine solche Lageranordnung vorgesehen sein.

Die Nabenstruktur 220 steht in Richtung des ersten Endes 201 von der Grundplatte 230 hervor. Eine von der Grundplatte 230 hervorstehende Länge I der Nabenstruktur 220 entlang der Rotationsachse 400 beträgt ungefähr die Hälfte einer Gesamtlänge L des Rotors 200 entlang der Rotationsachse 400. In weiteren Beispielen kann die hervorstehende Länge wenigstens ein Viertel, wenigstens ein Drittel, beispielsweise zwischen 35 Prozent und 40 Prozent oder zwischen 40 Prozent und 45 Prozent, oder wenigstens die Hälfte der Gesamtlänge betragen. Eine äußere Breite b der Nabenstruktur 220 senkrecht zur Rotationsachse 400 beträgt ungefähr die Hälfte einer Gesamtbreite B des Rotors 200 senkrecht zur Rotationsachse 400. In anderen Beispielen kann die äußere Breite wenigstens ein Drittel oder wenigstens die Hälfte der Gesamtbreite betragen.

Die Nabenstruktur 220 ist bezüglich der Rotationsachse 400 im Wesentlichen rotationssymmetrisch geformt. Die Nabenstruktur 220 ist im Wesentlichen zylindrisch geformt, wobei sich ein äußerer Querschnitt der Nabenstruktur 220 zum zweiten Ende 202 hin verjüngt und ein innerer Querschnitt der Nabenstruktur 220 sich zum zweiten Ende 202 hin aufweitet. Die Nabenstruktur kann in weiteren Beispielen beispielsweise wenigstens abschnittsweise im Wesentlichen konisch geformt sein oder einen entlang der Rotationsachse in anderer Weise variablen Querschnitt aufweisen. Die Nabenstruktur 220 weist einen oder mehrere Hohlräume 221 zum Aufnehmen des oder der einlassseitigen Rotorlagermagnete 211 auf.

Die Grundplatte 230 ist im Wesentlichen scheibenförmig in Form eines abgeflachten Zylinders. Die Grundplatte 230 umfasst einen oder mehrere Hohlräume 232 zum Aufnehmen des oder der auslassseitigen Rotorlagermagnete 213 und des oder der auslassseitigen Rotormotormagnete 212.

Die Beschaufelung 240 umfasst eine Mehrzahl von Schaufeln 243, wobei die Fluidkanäle 241 zwischen einander zugewandten jeweiligen ersten und zweiten Seitenflächen 244, 245 benachbarter Schaufeln 243 ausgebildet sind. Die Schaufeln 243 verlaufen spiralförmig radial von einem zentralen Bereich der Grundplatte 230 zu einem äußeren Bereich der Grundplatte 230 hin. Die Beschaufelung 240 ist somit eingerichtet, das geförderte Fluid im Betrieb mit einer radialen Strömungskomponente (in Richtung der Austrittsöffnung 242) zu beaufschlagen. Die Schaufeln 243 bzw. deren Seitenflächen 244, 245 stehen im Wesentlichen senkrecht zu der Grundplatte 230 und parallel zu der Rotationsachse 400, können aber alternativ in Bezug auf die Rotationsachse 400 angewinkelt und/oder gekrümmt sein.

Jeder der Fluidkanäle 241 weist eine den Fluidkanal 241 mit der Kavität 310 verbindende Durchlassöffnung 246 auf, die sich zwischen der Austrittsöffnung 242 und der Nabenstruktur 220 entlang einer dem ersten Ende 201 zugewandten Seite des Fluidkanals 241 erstreckt. Der Rotor 200 ist somit halboffen. Die Durchlassöffnung 246 jedes der Fluidkanäle 241 erstreckt sich entlang einer gesamten Ausdehnung des Fluidkanals 241 von der Austrittsöffnung 242 bis zu der Nabenstruktur 220. Es kann alternativ vorgesehen sein, dass sich die Durchlassöffnung 246 mindestens eines, vorzugsweise jedes, der Fluidkanäle 241 entlang nur eines Teils der Ausdehnung des Fluidkanals 241 von der Austrittsöffnung 242 in Richtung der Nabenstruktur 220 erstreckt. Es kann auch vorgesehen sein, dass nur einer oder mehrere, aber nicht alle Fluidkanäle 241 jeweils eine Durchlassöffnung 246 der beschriebenen Art aufweisen.

Die Nabenstruktur 220 umfasst einen entlang der Rotationsachse 400 verlaufenden und am ersten Ende 201 offenen Eintrittskanal 222, der mit den Fluidkanälen 241 innerhalb des Rotors 200 fluidisch verbunden ist. Die Nabenstruktur 220 wirkt somit als Fluideinlass des Rotors 200, durch den wenigstens ein Teil, insbesondere ein überwiegender Teil, des durch den Einlass 311 der Kavität 310 eintretende Fluid zu der Beschaufelung 240 geleitet wird. Die Fluidkanäle 241 weisen in den Eintrittskanal 222 mündende Eintrittsöffnungen 247 auf, durch die das Fluid im Betrieb von dem Eintrittskanal 222 in die Fluidkanäle 241 eintreten kann. Alternativ oder zusätzlich kann vorgesehen sein, dass das Fluid durch einen Zwischenraum zwischen der Nabenstruktur und einer Wand der Kavität in die Fluidkanäle eintritt. Ein Übergang 229 von der Beschaufelung 240 zu der Nabenstruktur 220 ist verrundet. Alternativ kann der Übergang ohne Verrundung ausgeführt sein.

Eine die Beschaufelung 240 umfassende erste Komponente 250 des Rotors 200 ist einstückig, insbesondere durch Fräsen aus einem zusammenhängenden Werkstoffstück, bevorzugt aus Metall, gebildet. Die erste Komponente 250 umfasst einen Teil der Grundplatte 230 und einen Teil der Nabenstruktur 220. Der Rotor 200 umfasst eine einstückig ausgeführte zweite Komponente 251, die einen am ersten Ende 201 angeordneten Teil der Nabenstruktur 220 umfasst, und eine einstückig ausgeführte dritte Komponente 252, die einen am zweiten Ende 202 angeordneten Teil der Grundplatte 230 umfasst. Der oder die Hohlräume 221 der Nabenstruktur sind zwischen der ersten Komponente 250 und der zweiten Komponente 251, der oder die Hohlräume 232 der Grundplatte zwischen der ersten Komponente 250 und der dritten Komponente 252 ausgebildet. Alternative Unterteilungen des Rotors 200 sind möglich, etwa kann die erste und/oder zweite und/oder dritte Komponente weiter in einstückige Komponenten oder Segmente unterteilt sein.

Die Fluidpumpe 100 kann eine Steuereinheit zum Steuern und/oder Regeln einer Rotation und/oder Position des Rotors 200 innerhalb der Kavität 310 umfassen und/oder mit einer solchen Steuereinheit verbindbar oder verbunden sein. Die Pumpe 100 kann einen oder mehrere Sensoren zum Bereitstellen von Messsignalen, entsprechend Parametern der Rotation und/oder Position des Rotors 200 und/oder entsprechend anderen Parametern, etwa einem Druck und/oder einer Druckdifferenz und/oder auf den Rotor 200 wirkenden Kräften und/oder Drehmomenten. Die Steuereinheit kann dazu eingerichtet sein, die Rotation und/oder Position des Rotors 200 basierend auf den Messsignalen zu steuern bzw. zu regeln. Die Steuereinheit kann beispielsweise dazu eingerichtet sein, ein an den Motorspulen 322 abgreifbares Messsignal mit einem von der Position und/oder Rotation des Rotors abhängigen Anteil zu erfassen; der Anteil kann insbesondere einer aufgrund der Rotation des Rotors 200 induzierten elektromotorischen Gegenkraft (Back Electromotive Force, Back-EMF) entsprechen. Ferner kann die Steuereinheit beispielsweise dazu eingerichtet sein, den Rotor 200 auf eine (insbesondere ortsveränderliche) Ziel-Lagerposition einzuregeln, an der auf den Rotor 200 wirkende äußere Kräfte entlang einer Lagerwirkrichtung, etwa entlang der Rotationsachse, sich zu einer vorgegebenen Kraft, insbesondere einer Nullkraft, addieren und/oder an der eine zum Ansteuern der Steuer- und/oder Motorspulen 321, 322 aufgewandte Leistung minimal ist.

FIG. 2, die sich nicht auf den Gegenstand der Anmeldung bezieht, zeigt einen geschlossenen Rotor 200'. Der geschlossene Rotor 200' gleicht dem in FIG. 1A-FIG. 1C gezeigten Rotor in wesentlichen Teilen; bei dem Rotor 200' sind allerdings die Fluidkanäle 241 zum ersten Ende 201 hin durch eine der Grundplatte 231 gegenüberliegende Deckplatte 260 begrenzt. Die Fluidkanäle 241 bilden damit im Inneren des Rotors 200' angeordnete Hohlräume. Die so gebildete komplexe Geometrie ist durch einstückige Fertigung, etwa durch Fräsen, nur schwer erhältlich. Zudem ist ein Zugang zu inneren Oberflächen erschwert, was etwa eine nachträgliche Oberflächenbearbeitung erschweren kann. Demgegenüber ermöglicht die Konstruktion des halboffenen Rotors 200 eine vereinfachte Fertigung wie oben beschrieben (insbesondere aufgrund der Durchlassöffnungen 246, also durch das Weglassen der Deckplatte 260).

Die in FIG. 3A und FIG. 3B gezeigten Kennlinien entsprechend gemessenen Daten, die mit Rotoren im Wesentlichen der in FIG. 1A-1C (halboffen) und FIG. 2 (geschlossen) gezeigten Arten erhalten wurden. Die Rotoren wurden jeweils in einer Fluidpumpe im Wesentlichen der in FIG. 1A gezeigten Art betrieben. Als Fluid wurde dabei Wasser verwendet.

FIG. 3A zeigt eine dp-Q-Kennlinie 501 eines halboffenen Rotors der in FIG. 1A bis FIG. 1C gezeigten Art (durchgezogener Graph) und eine dp-Q-Kennlinie eines geschlossenen Rotors der in FIG. 2 gezeigten Art (gestrichelter Graph). Die dp-Q-Kennlinie 501 des halboffenen Rotors weist über wesentliche Bereiche eine höhere Steilheit auf als die dp-Q-Kennlinie 502 des geschlossenen Rotors.

FIG. 3B zeigt eine Axialkraftkennlinie 503 eines halboffenen Rotors der in FIG. 1A bis FIG. 1C gezeigten Art (durchgezogener Graph) und eine Axialkraftkennlinie 504 eines geschlossenen Rotors der in FIG. 2 gezeigten Art (gestrichelter Graph). Die Axialkraftkennlinie 503 des halboffenen Rotors weist über wesentliche Bereiche eine höhere Steilheit auf als die Axialkraftkennlinie 504 des geschlossenen Rotors. Zudem verläuft die Axialkraftkennlinie 503 des halboffenen Rotors über weite Bereiche monoton, im Umfang der in diesen Bereichen gemessenen Datenpunkte insbesondere streng monoton, und ist also insbesondere umkehrbar. Dagegen weist die Axialkraftkennlinie 504 des geschlossenen Rotors einen Umkehrpunkt und nahezu flache (also nicht streng monotone) Bereiche auf und ist über weite Bereiche nicht umkehrbar.

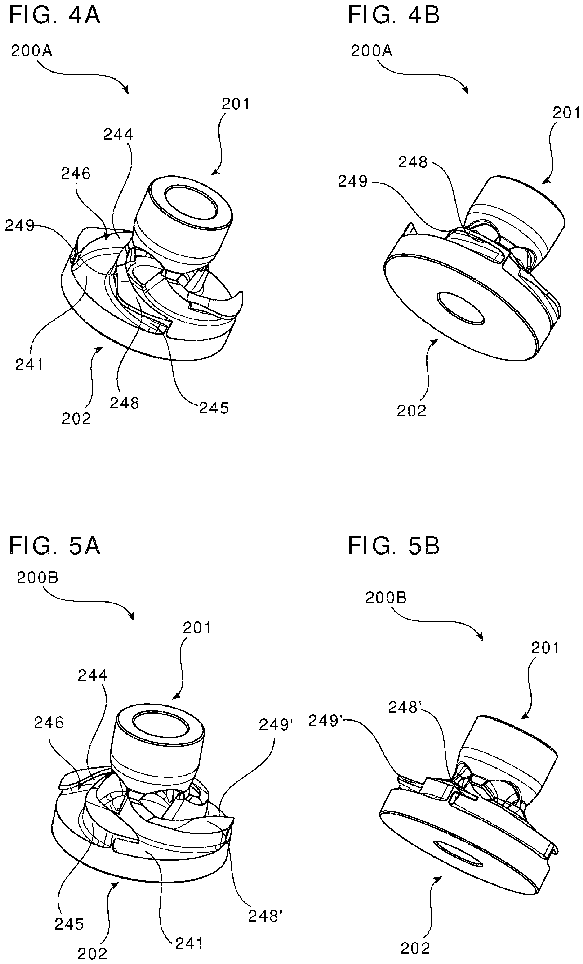

Der in FIG. 4A und FIG. 4B gezeigte Rotor 200A entspricht in wesentlichen Teilen dem in FIG. 1A bis FIG. 1C gezeigten Rotor 200. Zusätzlich zu dessen Merkmalen ist bei dem Rotor 200A jeder der Fluidkanäle 241 an seiner dem ersten Ende 201 zugewandten Seite auf Höhe der Durchlassöffnung 246 teilweise durch einen Vorsprung 248 (Winglet) der an den jeweiligen Fluidkanal 241 angrenzenden zweiten Seitenfläche 245 begrenzt. Der Vorsprung 248 kragt in einer Rotationsrichtung des Rotors 200A von der konvex gekrümmten zweiten Seitenfläche 245 aus. Der Vorsprung 248 weist an einem von der zweiten Seitenfläche 245 abgewandten Ende eine bereichsweise im Wesentlichen der Krümmung der zweiten Seitenfläche 245 folgende, aber beispielsweise bereichsweise stärker gekrümmte, Kante 249 auf. Die Kante 249 kann auch beispielsweise bereichsweise gerade und/oder bereichsweise schwächer als die zweite Seitenfläche 245 gekrümmt und/oder bereichsweise entgegen der Krümmung der zweiten Seitenfläche 245 gekrümmt sein. Der Vorsprung 248 kragt im Wesentlichen eben und senkrecht von der zweiten Seitenfläche 245 aus. Der Vorsprung 248 kann alternativ wenigstens bereichsweise gegenüber der zweiten Seitenfläche 245 abgewinkelt sein oder von der ebenen Form abweichen (also gekrümmt sein).

Auch der in FIG. 5A und FIG. 5B 200B gezeigte Rotor 200B entspricht in wesentlichen Teilen dem in FIG. 1A bis FIG. 1C gezeigten Rotor 200. Zusätzlich zu dessen Merkmalen ist bei dem Rotor 200B jeder der Fluidkanäle 241 an seiner dem ersten Ende 201 zugewandten Seite auf Höhe der Durchlassöffnung 246 teilweise durch einen Vorsprung 248' (Winglet) der an den jeweiligen Fluidkanal 241 angrenzenden ersten Seitenfläche 244 begrenzt. Im Unterschied zu dem in FIG. 4A und FIG. 4B gezeigten Rotor 200A kragt der Vorsprung 248' des Rotors 200B entgegen der Rotationsrichtung des Rotors 200B von der konkav gekrümmten ersten Seitenfläche 244 aus. Der Vorsprung 248' weist an einem von der ersten Seitenfläche 244 abgewandten Ende eine im Wesentlichen der Krümmung der ersten Seitenfläche 244 folgende, bereichsweise dieser Krümmung entgegengesetzt gekrümmte, Kante 249' auf. Die Kante 249' kann auch beispielsweise bereichsweise gerade und/oder bereichsweise schwächer als die erste Seitenfläche 244 gekrümmt und/oder bereichsweise stärker als die erste Seitenfläche 244 gekrümmt sein. Der Vorsprung 248' kragt im Wesentlichen eben und senkrecht von der ersten Seitenfläche 244 aus. Der Vorsprung 248' kann alternativ wenigstens bereichsweise gegenüber der ersten Seitenfläche 244 abgewinkelt sein oder von der ebenen Form abweichen (also gekrümmt sein).

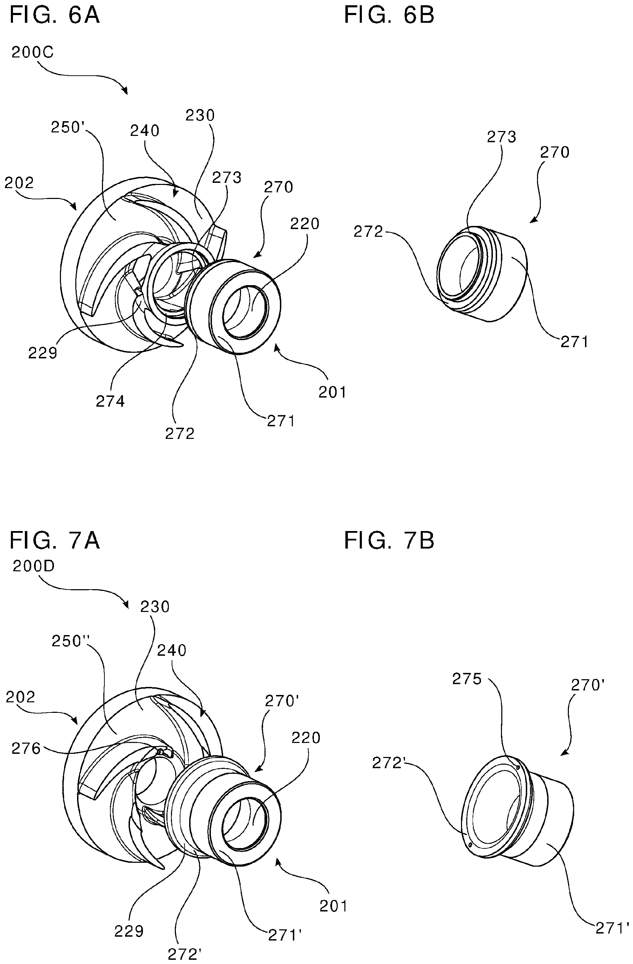

Der in FIG. 6A gezeigte Rotor 200C entspricht in wesentlichen Teilen dem in FIG. 1A bis FIG. 1C gezeigten Rotor 200, unterscheidet sich von diesem jedoch in der Unterteilung der Komponenten des Rotors 200C. Eine die Beschaufelung 240 umfassende erste Komponente 250' des Rotors 200C ist einstückig, insbesondere durch Fräsen aus einem zusammenhängenden Werkstoffstück aus Metall, gebildet. Die erste Komponente 250' umfasst wenigstens einen Teil der Grundplatte 230. Die Nabenstruktur 220 ist als separate Nabenbaugruppe 270 (separat aus einer anderen Perspektive gezeigt in FIG. 6B), umfassend eine an dem ersten Ende 201 angeordnete erste Nabenkomponente 271 und eine zwischen der ersten Nabenkomponente 271 und der Grundplatte 230 angeordnete zweite Nabenkomponente 272. Der oder die Hohlräume der Nabenstruktur zur Aufnahme von Magneten sind zwischen der ersten Nabenkomponente 271 und der zweiten Nabenkomponente 272 ausgebildet. Der verrundete Übergang 229 ist an der ersten Komponente 250' angeordnet.

Die zweite Nabenkomponente 272 umfasst einen dem zweiten Ende 202 zugewandten hervorstehenden Ausrichtring 273. Die erste Komponente 250' umfasst eine dem ersten Ende 201 zugewandte Ausrichtnut 274 zur Aufnahme des Ausrichtrings 273. Alternativ kann die erste Komponente 250' den Ausrichtring 273, die zweite Nabenkomponente 272 die Ausrichtnut 274 umfassen. Der Ausrichtring 273 und der Ausrichtnut 274 bilden eine Nutverbindung, mittels derer die erste Komponente 250' und die Nabenbaugruppe 270 miteinander verbindbar oder verbunden sind.

Der in FIG. 7A gezeigte Rotor 200D entspricht in wesentlichen Teilen dem in FIG. 1A bis FIG. 1C gezeigten Rotor 200, unterscheidet sich von diesem jedoch in der Unterteilung der Komponenten des Rotors 200D. Eine die Beschaufelung 240 umfassende erste Komponente 250" des Rotors 200C ist einstückig, insbesondere durch Fräsen aus einem zusammenhängenden Werkstoffstück aus Metall, gebildet. Die erste Komponente 250" umfasst wenigstens einen Teil der Grundplatte 230. Die Nabenstruktur 220 ist als separate Nabenbaugruppe 270' (separat aus einer anderen Perspektive gezeigt in FIG.7B), umfassend eine an dem ersten Ende 201 angeordnete erste Nabenkomponente 271' und eine zwischen der ersten Nabenkomponente 271' und der Grundplatte 230 angeordnete zweite Nabenkomponente 272'. Der oder die Hohlräume der Nabenstruktur zur Aufnahme von Magneten sind zwischen der ersten Nabenkomponente 271' und der zweiten Nabenkomponente 272' ausgebildet. Der verrundete Übergang 229 ist an der zweiten Nabenkomponente 272' angeordnet.

Die zweite Nabenkomponente 272' umfasst dem zweiten Ende 202 zugewandte Ausrichtlöcher 275. Die erste Komponente 250" umfasst dem ersten Ende 201 zugewandte Ausrichtstifte 276, die in jeweils entsprechende Ausrichtlöcher 275 aufnehmbar sind. Alternativ kann die erste Komponente 250" die Ausrichtlöcher 275, die zweite Nabenkomponente 272' die Ausrichtstifte 276 umfassen. Die Ausrichtlöcher 275 und die Ausrichtstifte 276 bilden eine Stiftverbindung, mittels derer die erste Komponente 250" und die Nabenbaugruppe 270' miteinander verbindbar oder verbunden sind.

### Liste der Bezugszeichen:

- 100: Fluidpumpe,
- 200, 200A, 200B, 200C, 200D,: halboffener Rotor,
- 200': geschlossener Rotor,
- 201: erstes Ende,
- 202: zweites Ende,
- 210: Rotormagnetanordnung,
- 211: einlassseitiger Rotorlagermagnet,
- 212: auslassseitiger Rotormotormagnet,
- 213: auslassseitiger Rotorlagermagnet,
- 220: Nabenstruktur,
- 221: Hohlraum,
- 222: Eintrittskanal,
- 229: Übergang,
- 230: Grundplatte,
- 231: zentrale Ausnehmung,
- 232: Hohlraum,
- 240: Beschaufelung,
- 241: Fluidkanal,
- 242: Austrittsöffnung,
- 243: Schaufel,
- 244: erste Seitenfläche,
- 245: zweite Seitenfläche,
- 246: Durchlassöffnung,
- 247: Eintrittsöffnung,
- 248, 248': Vorsprung,
- 249, 249': Kante,
- 250, 250', 250": erste Komponente,
- 251: zweite Komponente,
- 252: dritte Komponente,
- 260: Deckplatte,
- 270, 270': Nabenbaugruppe,
- 271, 271': erste Nabenkomponente,
- 272, 272': zweite Nabenkomponente,
- 273: Ausrichtring,
- 274: Ausrichtnut,
- 275: Ausrichtloch,
- 276: Ausrichtstift,
- 300: Stator,
- 301: Zapfen,
- 310: Kavität,
- 311: Einlass,
- 312: Auslass,
- 313: Volute,
- 320: Statormagnetlageranordnung,
- 321: Steuerspule,
- 322: Motorspule,
- 323: einlassseitiger Statorlagermagnet,
- 324: auslassseitiger Statorlagermagnet,
- 400: Rotationsachse,
- 501: dp-Q-Kennlinie, halboffener Rotor,
- 502: dp-Q-Kennlinie, geschlossener Rotor,
- 503: Axialkraftkennlinie, halboffener Rotor,
- 504: Axialkraftkennlinie, geschlossener Rotor,
- I: hervorstehende Länge,
- L: Gesamtlänge,
- b: äußere Breite,
- B: Gesamtbreite.

## Patentansprüche

1. Rotor (200, 200A, 200B, 200C, 200D) für eine Fluidpumpe (100) zur Herzunterstützung, umfassend
eine Rotormagnetanordnung (210) zum berührungsfreien Lagern des Rotors (200, 200A, 200B, 200C, 200D) innerhalb einer Kavität (310) der Fluidpumpe (100) derart, dass der Rotor (200, 200A, 200B, 200C, 200D) zum Fördern eines Fluid von einem Einlass (311) der Kavität (310) zu einem Auslass (312) der Kavität (310) um eine Rotationsachse (400) drehbar ist und ein erstes Ende (201) des Rotors (200, 200A, 200B, 200C, 200D) näher an dem Einlass (311) angeordnet ist als ein dem ersten Ende (201) entlang der Rotationsachse (400) entgegengesetztes zweites Ende (202) des Rotors (200, 200A, 200B, 200C, 200D),
eine am ersten Ende (201) angeordnete, um die Rotationsachse (400) zentrierte Nabenstruktur (220), die einen oder mehrere Magnete der Rotormagnetanordnung (210) umfasst,
eine am zweiten Ende (202) oder zwischen der Nabenstruktur (220) und dem zweiten Ende (202) angeordnete Grundplatte (230) mit einer Beschaufelung (240) zum Fördern des Fluids, wobei Zwischenräume der Beschaufelung (240) Fluidkanäle (241) bilden, die in jeweilige Austrittsöffnungen (242) münden und zum zweiten Ende (202) hin durch die Grundplatte (230) begrenzt sind,
wobei mindestens einer, vorzugsweise jeder, der Fluidkanäle (241) eine den Fluidkanal (241) mit der Kavität (310) verbindende Durchlassöffnung (246) aufweist, die sich zwischen der Austrittsöffnung (242) und der Nabenstruktur (220) wenigstens bereichsweise entlang einer dem ersten Ende (201) zugewandten ersten Seite des Fluidkanals (241) erstreckt.

2. Rotor (200, 200A, 200B, 200C, 200D) nach Anspruch 1, wobei sich die Durchlassöffnung (246) mindestens eines, vorzugsweise jedes, der Fluidkanäle (241) entlang wenigstens eines Teils einer Ausdehnung des Fluidkanals (241) von der Austrittsöffnung (242) in Richtung der Nabenstruktur (220) erstreckt.

3. Rotor (200, 200A, 200B, 200C, 200D) nach Anspruch 2, wobei sich die Durchlassöffnung (246) mindestens eines, vorzugsweise jedes, der Fluidkanäle (241), entlang der gesamten Ausdehnung des Fluidkanals (241) von der Austrittsöffnung (242) bis zu der Nabenstruktur (220) erstreckt.

4. Rotor (200, 200A, 200B, 200C, 200D) nach einem der vorhergehenden Ansprüche, wobei mindestens einer, vorzugsweise jeder, der Fluidkanäle (241) an der ersten Seite auf Höhe der Durchlassöffnung (246) teilweise durch einen Vorsprung (248, 248') einer an den jeweiligen Fluidkanal (241) angrenzenden Fläche der Beschaufelung (240) begrenzt ist.

5. Rotor (200, 200A, 200B, 200C, 200D) nach Anspruch 4, wobei der Vorsprung (248, 248') entgegen einer Rotationsrichtung des Rotors (200, 200A, 200B, 200C, 200D) von der an den jeweiligen Fluidkanal (241) angrenzenden Fläche der Beschaufelung (240) auskragt und/oder diese Fläche eine konkave Krümmung aufweist.

6. Rotor (200, 200A, 200B, 200C, 200D) nach Anspruch 4, wobei der Vorsprung (248, 248') in einer Rotationsrichtung des Rotors (200, 200A, 200B, 200C, 200D) von der an den jeweiligen Fluidkanal (241) angrenzenden Fläche der Beschaufelung (240) auskragt und/oder diese Fläche eine konvexe Krümmung aufweist.

7. Rotor (200, 200A, 200B, 200C, 200D) nach einem der vorhergehenden Ansprüche, wobei die Nabenstruktur (220) einen entlang der Rotationsachse (400) verlaufenden und am ersten Ende (201) offenen Eintrittskanal (222) umfasst, der mit den Fluidkanälen (241) innerhalb des Rotors (200, 200A, 200B, 200C, 200D) fluidisch verbunden ist.

8. Rotor (200, 200A, 200B, 200C, 200D) nach einem der vorhergehenden Ansprüche, wobei ein Übergang (229) von der Beschaufelung (240) zu der Nabenstruktur (220) verrundet ist.

9. Rotor (200, 200A, 200B, 200C, 200D) nach einem der vorhergehenden Ansprüche, wobei eine die Beschaufelung (240) umfassende Komponente (250, 250', 250") des Rotors (200, 200A, 200B, 200C, 200D) einstückig, insbesondere durch Fräsen aus einem zusammenhängenden Werkstoffstück, gebildet ist.

10. Rotor (200, 200A, 200B, 200C, 200D) nach Anspruch 9, wobei die die Beschaufelung (240) umfassende einstückig gebildete Komponente (250, 250', 250") wenigstens einen Teil der Grundplatte (230) und/oder wenigstens einen Teil der Nabenstruktur (220) umfasst.

11. Rotor (200, 200A, 200B, 200C, 200D) nach einem der vorhergehenden Ansprüche, wobei der Rotor (200, 200A, 200B, 200C, 200D) aus einer ersten Komponente, umfassend wenigstens einen Teil der Nabenstruktur, und mindestens einer zweiten Komponente zusammengesetzt oder zusammensetzbar ist.

12. Rotor (200, 200A, 200B, 200C, 200D) nach einem der vorhergehenden Ansprüche, wobei die Grundplatte (230) einen oder mehrere Magnete der Rotormagnetanordnung (210) umfasst.

13. Rotor (200, 200A, 200B, 200C, 200D) nach einem der vorhergehenden Ansprüche, wobei die Grundplatte (230) eine zentrale Ausnehmung (231) zur berührungsfreien Aufnahme eines Teils eines Stators der Fluidpumpe (100), insbesondere eines Zapfens mit einer Magnetlagerkomponente, umfasst.

14. Fluidpumpe (100) zur Herzunterstützung, umfassend
einen Rotor (200, 200A, 200B, 200C, 200D) nach einem der vorhergehenden Ansprüche,
einen Stator (300) mit einer Kavität (310) zur Aufnahme des Rotors (200, 200A, 200B, 200C, 200D) und einer Statormagnetlageranordnung (320), die zusammen mit der Rotormagnetanordnung (210) ein Magnetlager, insbesondere ein aktives Magnetlager, zum berührungsfreien Lagern des Rotors (200, 200A, 200B, 200C, 200D) innerhalb der Kavität (310) bildet.

15. Fluidpumpe (100) nach Anspruch 14, wobei die Fluidpumpe (100) eine Zentrifugalpumpe ist und/oder wobei der Stator (300) und der Rotor (200, 200A, 200B, 200C, 200D) einen Axialflussmotor bilden.
